# EUROPEAN PATENT APPLICATION

(11) **EP 4 541 889 A1**
(43) Date of publication of application: **23.04.2025**
(21) Application number: 23826756.1
(22) Date of filing: 30.03.2023
(51) Int. Cl.: C12N 15/11, C12Q 1/6811, C12Q 1/6818

(54) **SINGLE-STRANDED OLIGONUCLEOTIDE LABELED BY FLUOROPHORE AND QUENCHER**

(30) Priority: 20.06.2022 JP 2022099034
(71) Applicant: Toppan Holdings Inc., Tokyo 110-0016 (JP)
(72) Inventor: MAKINO, Yoichi, Tokyo 110-0016 (JP); OGINO, Masayuki, Tokyo 110-0016 (JP); NAGAHARA, Misaki, Tokyo 110-0016 (JP); HORIUCHI, Yosuke, Tokyo 110-0016 (JP); SUZUKI, Yuta, Tokyo 110-0016 (JP)
(74) Representative: Plasseraud IP
(86) International application number: PCT/JP2023/013078
(87) International publication number: WO 2023/248567

(57) **Abstract**

A single-stranded oligonucleotide labeled with a fluorophore and a quencher, wherein a hairpin structure is formed on the 5'-side by self-hybridization, when a single-stranded oligonucleotide having a complementary base sequence on the 3'-side hybridizes, a triple-stranded structure is formed at the 5'-end, the fluorophore and the quencher are released by cleavage by a flap endonuclease that recognizes the triple-stranded structure, emitting fluorescence upon irradiation with excitation light, the hairpin structure internally contains a non-complementary base pair, and the quencher is bound to a base of a nucleotide residue.

## Description

### [Technical Field]

The present invention relates to a single-stranded oligonucleotide labeled with a fluorophore and a quencher. The present application claims the benefit of a priority from Japanese Patent Application No. 2022-099034 filed in Japan on June 20, 2022, the contents of which are incorporated herein by reference.

### [Background Art]

A method using fluorescence resonance energy transfer (FRET) is known as a method for detecting trace amounts of molecules to be analyzed.

For example, in genetic diagnosis, there are many methods of accurately and promptly detecting and/or quantifying target nucleic acids. Among these methods, an invasive cleavage assay (ICA) is excellent in operability and reaction stability (see, for example, NPL 1).

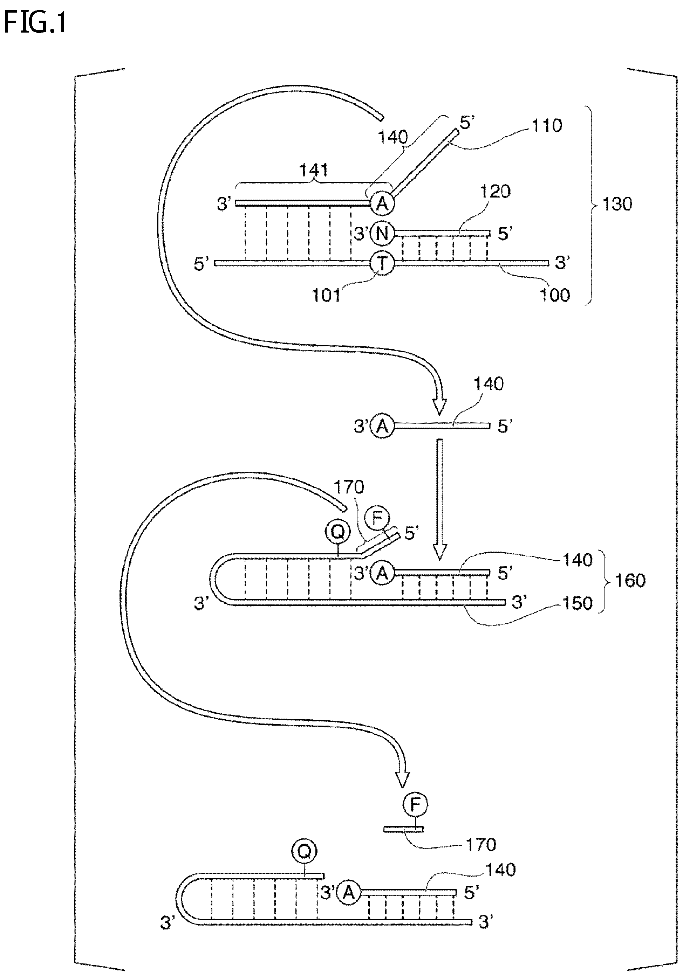

ICA is explained here with reference to Fig. 1. Fig. 1 is a schematic view illustrating an example of ICA. In the example of Fig. 1, the presence of a T (thymine) base 101 in a target nucleic acid 100 is detected. First, the target nucleic acid 100 is hybridized with a complementary flap probe 110 and an invader probe 120. As a result, the invader probe 120 hybridizes to a site of the target nucleic acid 100 that is adjacent to the location where the flap probe 110 hybridizes. Then, at least one base at the 3'-end of the invader probe 120 penetrates the 5'-end of a region 141 where the flap probe 110 hybridizes to the target nucleic acid 100, thus forming a first triple-stranded structure 130.

Subsequently, when the first triple-stranded structure 130 is reacted with a flap endonuclease, a flap site 140 of the first triple-stranded structure 130 is cleaved to produce a nucleic acid fragment 140. Subsequently, the nucleic acid fragment 140 hybridizes to a nucleic acid fragment 150 to form a second triple-stranded structure 160.

In the example of Fig. 1, a fluorophore F is bound to the 5'-end of the nucleic acid fragment 150, and a quencher Q is bound to the 3'-side by a few bases from the 5'-end of the nucleic acid fragment 150. The fluorophore F and the quencher Q are located in the spatial neighborhood. Accordingly, the fluorescence emitted by the fluorophore F is quenched by the quencher Q.

Subsequently, when the second triple-stranded structure 160 is reacted with a flap endonuclease, a flap site 170 of the second triple-stranded structure 160 is cleaved to produce a nucleic acid fragment 170. As a result, the fluorophore F is released from the quencher Q and emits fluorescence upon irradiation with excitation light. By detecting the emitted fluorescence, the presence of the T (thymine) base 101 in the target nucleic acid 100 can be detected.

### [Citation List]

### [Patent Literature]

PTL 1: JP 2004-309405 A

### [Non-Patent Literature]

NPL 1: Eis P. S. et al., An invasive cleavage assay for direct quantitation of specific RNAs, Nature Biotechnology, 19 (7), 673-676, 2001.

### [Summary of the Invention]

### [Technical Problem]

The inventors have found that in the synthesis of a single-stranded oligonucleotide labeled with a fluorophore and a quencher, synthesis efficiency was low in some cases. An object of the present invention is to provide a technique for improving the synthesis efficiency of a single-stranded oligonucleotide labeled with a fluorophore and a quencher.

### [Solution to Problem]

The present invention includes the following aspects.
[1] A single-stranded oligonucleotide labeled with a fluorophore and a quencher, wherein a hairpin structure is formed on the 5'-side by self-hybridization, when a single-stranded oligonucleotide having a complementary base sequence on the 3'-side hybridizes, a triple-stranded structure is formed at the 5'-end, the fluorophore and the quencher are released by cleavage by a flap endonuclease that recognizes the triple-stranded structure, emitting fluorescence upon irradiation with excitation light, the hairpin structure internally contains a non-complementary base pair, and the quencher is bound to a base of a nucleotide residue.
[2] The single-stranded oligonucleotide according to claim 1, wherein the fluorophore is labeled to a nucleotide residue at the 5'-end, and the quencher is labeled to, among nucleotide residues that constitute the non-complementary base pair, a nucleotide residue closer to the 5'-side.
[3] The single-stranded oligonucleotide according to [1] or [2], wherein the base of the nucleotide residue at the 5'-end is a pyrimidine base.
[4] The single-stranded oligonucleotide according to any one of [1] to [3], wherein the quencher is labeled closer to the 3'-side than the fluorophore, and an oligonucleotide fragment containing the fluorophore is released upon cleavage by a flap endonuclease.
[5] A method for improving the synthesis efficiency of a single-stranded oligonucleotide labelled with a fluorophore and a quencher, the method comprising synthesizing, as the single-stranded oligonucleotide, a single-stranded oligonucleotide labeled with a fluorophore and a quencher, wherein a hairpin structure is formed on the 5'-side by self-hybridization, when a single-stranded oligonucleotide having a complementary base sequence on the 3'-side hybridizes, a triple-stranded structure is formed at the 5'-end, the fluorophore and the quencher are released by cleavage by a flap endonuclease that recognizes the triple-stranded structure, emitting fluorescence upon irradiation with excitation light, and the hairpin structure internally contains a non-complementary base pair.
[6] The method according to [5], which synthesizes, as the single-stranded oligonucleotide, a single-stranded oligonucleotide in which the quencher is bound to a base of a nucleotide residue.

### [Advantageous Effects of the Invention]

The present invention makes it possible to provide a technique for improving the synthesis efficiency of a single-stranded oligonucleotide labeled with a fluorophore and a quencher.

### [Brief Description of the Drawing]

[Fig. 1] Fig. 1 is a schematic view illustrating an example of an invasive cleavage assay (ICA).

### [Description of the Embodiments]

### [Single-stranded oligonucleotide]

In an embodiment, the present invention provides a single-stranded oligonucleotide labeled with a fluorophore and a quencher, wherein a hairpin structure is formed on the 5'-side by self-hybridization, when a single-stranded oligonucleotide having a complementary base sequence on the 3'-side hybridizes, a triple-stranded structure is formed at the 5'-end, the fluorophore and the quencher are released by cleavage by a flap endonuclease that recognizes the triple-stranded structure, emitting fluorescence upon irradiation with excitation light, the hairpin structure internally contains a non-complementary base pair, and the quencher is bound to a base of a nucleotide residue.

The single-stranded oligonucleotide of the present embodiment is a fluorescent substrate for ICA, and can also be regarded as a flap endonuclease substrate. Examples of the flap endonuclease include flap endonuclease 1 (NCBI Accession Number: WP_011012561.1, Holliday junction 5' flap endonuclease (GEN1) (NCBI Accession Number: NP_001123481.3), excision repair protein (NCBI Accession Number: AAC37533.1, and the like.

The triple-stranded structure formed at the 5'-end when a hairpin structure is formed on the 5'-side by self-hybridization and a single-stranded oligonucleotide having a complementary base sequence on the 3'-side hybridizes corresponds to the second triple-stranded structure 160 shown in Fig. 1. That is, the single-stranded oligonucleotide having a complementary base sequence on the 3'-side corresponds to the nucleic acid fragment 140 derived from the flap site 140.

As described below in the examples, because the hairpin structure internally contains a non-complementary base pair, the synthesis efficiency of a single-stranded oligonucleotide labeled with a fluorophore and a quencher can be significantly improved compared to when the hairpin structure does not internally contain a non-complementary base pair.

Because the single-stranded oligonucleotide of the present embodiment has a self-complementary sequence, a stem-loop structure is formed as the synthesis progresses. Therefore, the reaction site for elongation of chemical synthesis becomes bulky, and steric hindrance is considered to inhibit nucleotide elongation. Therefore, it is considered that by introducing one or several mismatch bases into the self-complementary sequence portion (inside the hairpin structure), it is possible to alleviate the steric hindrance and suppress the inhibition of the elongation of chemical synthesis. As a result, it is considered that the efficiency of oligonucleotide synthesis can be improved.

**In** the single-stranded oligonucleotide of the present embodiment, the number of non-complementary base pairs may be 1 to 3, 1 or 2, or 1.

**In** the single-stranded oligonucleotide of the present embodiment, the quencher is bound to the base of a nucleotide residue. As described below in the examples, because a quencher is bound to the base of a nucleotide residue, the synthesis efficiency of a single-stranded oligonucleotide labeled with a fluorophore and a quencher can be significantly improved compared to when a quencher is bound to a part other than the base of a nucleotide residue. The inventors assume that the binding of a quencher to the base moiety alleviates the steric hindrance, improving synthesis efficiency.

The single-stranded oligonucleotide of the present embodiment preferably has a length of about 20 to 150 bases. In addition, the number of base pairs, which form the hairpin structure, is preferably about 5 to 50.

### (Fluorophore)

The fluorophore is not particularly limited, and examples include fluorescein, ATTO 425, ATTO 488, Alexa 488, ATTO 542, Yakima Y, Redmond R, ATTO 643, Alexa 647, Alexa 680, Alexa 568, FAM, ATTO 633, Cy5, HiLyte Fluor 647, ATTO 663, and the like.

It is preferable for the single-stranded oligonucleotide of the present embodiment that the nucleotide residue at the 5'-end is labeled with a fluorophore, and that among nucleotide residues that constitute a non-complementary base pair, a nucleotide residue closer to the 5'-side is labeled with a quencher.

As described below in the examples, such a fluorescent substrate can be synthesized with high synthesis efficiency. In the present specification, the phrase "high synthesis efficiency" can also be rephrased as high yield. For example, this phrase means that when solid-phase synthesis is performed in a 1 mol column by the phosphoramidite method, 90 pmol or more of the target single-stranded oligonucleotide can be synthesized.

### (Quencher)

The quencher is not particularly limited as long as it can quench the fluorescence of the fluorophore used. Examples include Black Hole Quencher (BHQ) (registered trademark)-1, BHQ (registered trademark)-2, BHQ (registered trademark)-3, Tide Quencher 1 (TQ1), Tide Quencher 2 (TQ2), Tide Quencher 2WS (TQ2WS), Tide Quencher 3 (TQ3), Tide Quencher 3WS (TQ3WS), Tide Quencher 4 (TQ4), Tide Quencher 4WS (TQ4WS), Tide Quencher 5 (TQ5), Tide Quencher 5WS (TQ5WS), Tide Quencher 6WS (TQ6WS), Tide Quencher 7WS (TQ7WS), QSY35, QSY7, QSY9, QSY21, Iowa Black FQ, Iowa Black RQ, and the like. As the quencher, one that can quench the fluorescence of the fluorophore used is selected.

When the fluorophore is in the spatial neighborhood of the quencher, the fluorescence from the fluorophore is quenched by the quencher. "Quenching fluorescence" means the following. When the fluorophore is irradiated with excitation light in the absence of a quencher, the intensity of fluorescence emitted from the fluorophore is denoted by A. Further, when the fluorophore is irradiated with excitation light in the presence of a quencher in the spatial neighborhood of the fluorophore, the intensity of fluorescence emitted from the fluorophore is denoted by B. In this context, "quenching fluorescence" means that the value of B/A is 40% or less.

When the fluorophore is in the spatial neighborhood of the quencher, the distance between the fluorophore and the quencher is not particularly limited as long as the fluorescence emitted from the fluorophore is quenched by the quencher. The distance is preferably 10 nm or less, more preferably 5 nm or less, and even more preferably 2 nm or less.

In the single-stranded oligonucleotide of the present embodiment, the quencher is bound to the base of a nucleotide residue. More specific examples of the quencher bound to the base of a nucleotide residue includes a quencher represented by the following formula (1). The quencher of the following formula (1) is BHQ (registered trademark)-1.

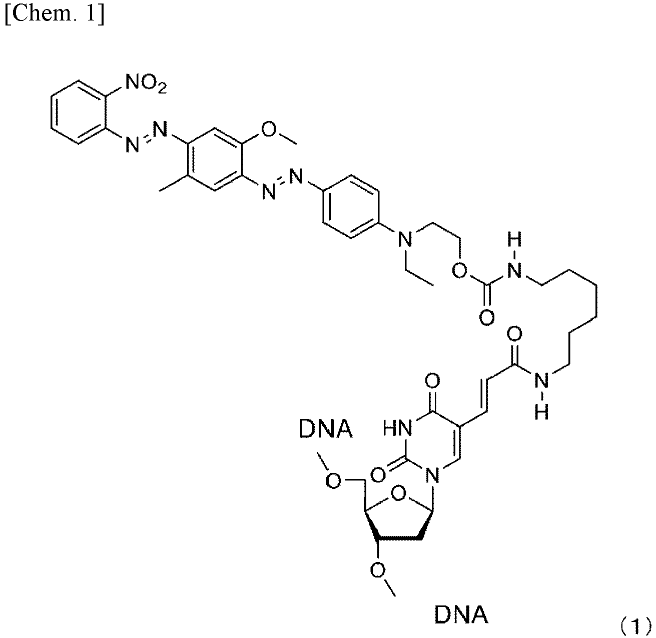

### (Oligonucleotide)

The single-stranded oligonucleotide that forms the fluorescent substrate of the present embodiment is not particularly limited as long as the effects of the present invention are exhibited, and the single-stranded oligonucleotide may be a natural nucleic acid or a synthetic nucleic acid.

Examples of the natural nucleic acid include genomic DNA, mRNA, rRNA, hnRNA, miRNA, tRNA, and the like. The natural nucleic acid may be recovered from a living organism, or from water, organic matter, or the like that has been in contact with a living organism. Examples of the method for recovering the natural nucleic acid include known methods, such as the phenol/chloroform method.

Examples of the synthetic nucleic acid include synthetic DNA, synthetic RNA, cDNA, bridged nucleic acid (BNA), locked nucleic acid (LNA), and the like.

The nucleic acid synthesis method is not particularly limited, and examples include known chemical synthesis methods such as solid-phase synthesis, known nucleic acid amplification methods, reverse transcription reactions, and the like. Examples of the nucleic acid amplification method include PCR, LAMP, SMAP, NASBA, and RCA. Among these, it is preferable to synthesize the single-stranded oligonucleotide of the present embodiment by solid-phase synthesis.

Solid-phase synthesis can be performed by repeating a first step of removing, from a nucleotide derivative having a protecting group or an oligonucleotide derivative having a protecting group bound to a solid phase, the protecting group to generate a nucleotide or an oligonucleotide, and a second step of binding a nucleotide derivative having a protecting group to the nucleotide or oligonucleotide.

The protecting group is not particularly limited, and examples include a dimethoxytrityl group (DMT), an acetyl group (Ac); a benzoyl group (Bz); a trityl group (Tr), a monomethoxytrityl group (MMT), a trimethoxytrityl group (TMT), and other ether protecting groups; β-methoxyethoxymethyl ether (MEM), a methoxymethyl ether group (MOM), a tetrahydropyranyl group (THP), and other acetal protecting groups; and a t-butyldimethylsilyl group (TBS) and other silyl ether groups. These protecting groups are used when the functional group to be protected is a hydroxyl group. When the functional group to be protected is an amino group or the like, a suitable protecting group can be selected and used as appropriate.

The protecting groups may be used singly or in combination of two or more. For example, a nucleotide derivative having a protecting group with a different chemical structure may be used each time a nucleotide derivative is bound.

The nucleotide derivative having a protecting group may be one that is used for general nucleic acid synthesis methods. An example of such a nucleic acid synthesis method is the phosphoramidite method, and a phosphoramidized nucleotide derivative can be used as the nucleotide derivative. Further, examples of the protecting group of the nucleotide derivative include those described above.

As the solid phase, for example, beads may be used. Examples of the material of the solid phase include silicone, glass, quartz, soda lime glass, polyamide resin, and the like.

Examples of the functional group to be protected with a protecting group include, but are not limited to, a hydroxyl group bound to the carbon at position 5 of ribose or deoxyribose. Examples of the nucleotide derivative include, but are not limited to, DMT-dA phosphoramidite, DMT-dT phosphoramidite, DMT-dG phosphoramidite, DMT-dC phosphoramidite, and the like. The nucleotide derivative may be a product commercially available for nucleic acid synthesis. The nucleotides from which the nucleotide derivative is derived may be RNA, or may be an artificial nucleic acid, such as BNA (bridged nucleic acid) or PNA (peptide nucleic acid).

The use of a fluorophore-binding nucleotide derivative or a quencher-binding nucleotide derivative as the nucleotide derivative allows the synthesis of a single-stranded oligonucleotide labeled with a fluorophore and a quencher.

As described above, solid-phase synthesis can be performed by repeating a first step of removing, from a nucleotide derivative having a protecting group or an oligonucleotide derivative having a protecting group bound to a solid phase, the protecting group to generate a nucleotide or an oligonucleotide, and a second step of binding a nucleotide derivative having a protecting group to the nucleotide or oligonucleotide.

The nucleotide derivative having a protecting group or the oligonucleotide derivative having a protecting group refers to an oligonucleotide under synthesis and having a protecting group at the end. The protecting group is the same as the one described above, and may be, for example, an acid-degradable protecting group that is deprotected by the action of acid.

In the first step, for example, acid is applied to an oligonucleotide derivative having a protecting group to remove the protecting group and form a hydroxyl group at the 5'-end of the oligonucleotide. For example, when the protecting group is a DMT group, the removed protecting group is a DMT cation.

In the second step, a nucleotide derivative having a protecting group is bound to the deprotected oligonucleotide to extend the nucleotide chain, and an oligonucleotide derivative having a protecting group at the end is formed again.

The second step may further have a step of capping unreacted functional groups by acetylation or other means so that they are not involved in the subsequent cycles, or a step of oxidizing the bond between the oligonucleotide and the terminal nucleotide derivative to convert trivalent phosphorus to pentavalent phosphate. The oligonucleotide can be elongated by repeating the first and second steps.

It is preferable for the single-stranded oligonucleotide of the present embodiment that a fluorophore is located near the nucleotide residue at the 5'-end, and that the base of the nucleotide residue at the 5'-end is a pyrimidine base. Purine bases tend to quench the fluorescence of fluorophores. Therefore, when the base of the nucleotide residue at the 5'-end is a pyrimidine base, quenching of the fluorescence of fluorophores is suppressed, which is preferable.

### [Method for improving the synthesis efficiency of a single-stranded oligonucleotide labeled with a fluorophore and a quencher]

In an embodiment, the present invention provides a method for improving the synthesis efficiency of a single-stranded oligonucleotide labeled with a fluorophore and a quencher, comprising synthesizing, as the single-stranded oligonucleotide, a single-stranded oligonucleotide having a predetermined structure.

In the method of the present embodiment, in the single-stranded oligonucleotide, a hairpin structure is formed on the 5'-side by self-hybridization, when a single-stranded oligonucleotide having a complementary base sequence on the 3'-side hybridizes, a triple-stranded structure is formed at the 5'-end, the fluorophore and the quencher are released by cleavage by a flap endonuclease that recognizes the triple-stranded structure, emitting fluorescence upon irradiation with excitation light, the hairpin structure internally contains a non-complementary base pair, and the quencher is bound to a base of a nucleotide residue.

As described below in the examples, the inventors revealed that the synthesis efficiency of a single-stranded oligonucleotide can be improved by synthesizing one having the above structure.

In the method of the present embodiment, the fluorophore, the quencher, the oligonucleotide, the oligonucleotide synthesis method, and the like are the same as those described above.

It is preferable for the single-stranded oligonucleotide that the nucleotide residue at the 5'-end is labeled with a fluorophore, and that among nucleotide residues that constitute a non-complementary base pair, a nucleotide residue closer to the 5'-side is labeled with a quencher.

It is preferable for the single-stranded oligonucleotide that a fluorophore is located near the nucleotide residue at the 5'-end, and that the base of the nucleotide residue at the 5'-end is a pyrimidine base.

It is preferable for the single-stranded oligonucleotide that the quencher is labeled closer to the 3'-side than the fluorophore, and that an oligonucleotide fragment containing the fluorophore is released upon cleavage by a flap endonuclease.

### [Examples]

### [Experimental Example 1]

### (Consideration of fluorescent substrate)

The single-stranded oligonucleotides shown in Table 1 below were synthesized by solid-phase synthesis using the phosphoramidite method, and their yields were compared.

**[Table 1]**

| Name | SEQ. ID. No. | Base sequence (5'→3') |
|---|---|---|
| Fluorescent substrate 1 | 1 | F-TCT-T(Q)-AGCCGGTTTTCCGGCTAAGACCTCGGCGCG |
| Fluorescent substrate 2 | 2 | F-TCT-T(Q)-AGCCGGTTTTCCGGCTGAGACCTCGGCGCG |

The fluorescent substrate 1 (SEQ ID No: 1) shown in Table 1 above is a fluorescent substrate that does not contain a non-complementary base pair within its hairpin structure, and specifically has a structure as shown in the following formula (2). F in the fluorescent substrate 1 represents ATTO488, and Q represents BHQ (registered trademark)-1. As shown in the following formula (2), the fluorescent substrate 1 does not contain a non-complementary base pair within its hairpin structure. Further, in the fluorescent substrate 1, the quencher was bound to the base of a nucleotide residue.

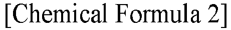

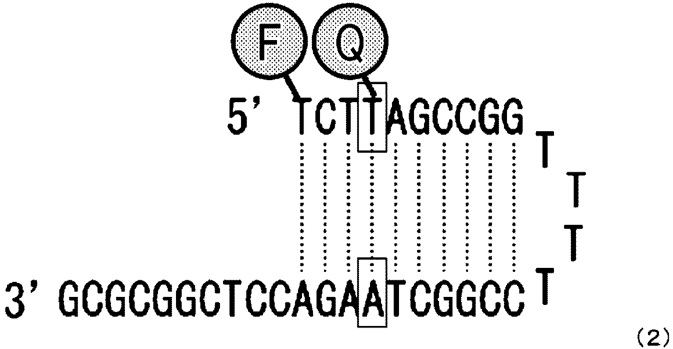

The fluorescent substrate 2 (SEQ ID No: 2) shown in Table 1 above is a fluorescent substrate that contains a non-complementary base pair within its hairpin structure, and specifically has a structure as shown in the following formula (3). F in the fluorescent substrate 2 represents ATTO488, and Q represents BHQ (registered trademark)-1. As shown in the following formula (3), the fluorescent substrate 2 contains a non-complementary base pair within its hairpin structure. In the following formula (3), the T (thymine) and G (guanine) residues surrounded by squares are not complementary to each other. Further, in the fluorescent substrate 2, the quencher was bound to the base of a nucleotide residue.

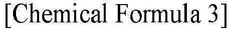

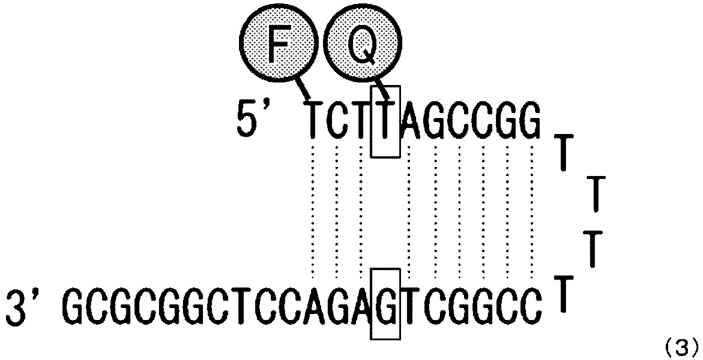

In solid-phase synthesis, a 1 mol column was used. Table 2 below shows the yield of each single-stranded oligonucleotide.

**[Table 2]**

| Name | SEQ. ID. No. | Yield (pmol) |
|---|---|---|
| Fluorescent substrate 1 | 1 | 71 |
| Fluorescent substrate 2 | 2 | 93 |

As a result, it was revealed that the yield of the fluorescent substrate 2, which contained a non-complementary base pair within its hairpin structure and in which the quencher was bound to the base of a nucleotide residue, was significantly improved in comparison with the fluorescent substrate 1.

### [Industrial Applicability]

The present invention makes it possible to provide a technique for improving the synthesis efficiency of a single-stranded oligonucleotide labeled with a fluorophore and a quencher.

### [Reference Signs List]

- 100: Target nucleic acid,
- 101: base,
- 110: flap probe,
- 120: Invader probe,
- 130: first triple-stranded structure,
- 140, 170: flap sites (nucleic acid fragments),
- 141: region,
- 150: nucleic acid fragment,
- 151: mismatch site,
- 160: second triple-stranded structure,
- F: fluorophore,
- Q: quencher.

## Claims

1. A single-stranded oligonucleotide labeled with a fluorophore and a quencher, wherein a hairpin structure is formed on the 5'-side by self-hybridization, when a single-stranded oligonucleotide having a complementary base sequence on the 3'-side hybridizes, a triple-stranded structure is formed at the 5'-end, the fluorophore and the quencher are released by cleavage by a flap endonuclease that recognizes the triple-stranded structure, emitting fluorescence upon irradiation with excitation light, the hairpin structure internally contains a non-complementary base pair, and the quencher is bound to a base of a nucleotide residue.

2. The single-stranded oligonucleotide according to claim **1,** wherein the fluorophore is labeled to a nucleotide residue at the 5'-end, and the quencher is labeled to, among nucleotide residues that constitute the non-complementary base pair, a nucleotide residue closer to the 5'-side.

3. The single-stranded oligonucleotide according to claim 1 or 2, wherein the base of the nucleotide residue at the 5'-end is a pyrimidine base.

4. The single-stranded oligonucleotide according to claim 1 or 2, wherein the quencher is labeled closer to the 3'-side than the fluorophore, and an oligonucleotide fragment containing the fluorophore is released upon cleavage by a flap endonuclease.

5. A method for improving the synthesis efficiency of a single-stranded oligonucleotide labelled with a fluorophore and a quencher, the method comprising synthesizing, as the single-stranded oligonucleotide, a single-stranded oligonucleotide labeled with a fluorophore and a quencher, wherein a hairpin structure is formed on the 5'-side by self-hybridization, when a single-stranded oligonucleotide having a complementary base sequence on the 3'-side hybridizes, a triple-stranded structure is formed at the 5'-end, the fluorophore and the quencher are released by cleavage by a flap endonuclease that recognizes the triple-stranded structure, emitting fluorescence upon irradiation with excitation light, and the hairpin structure internally contains a non-complementary base pair.

6. The method according to claim 5, which synthesizes, as the single-stranded oligonucleotide, a single-stranded oligonucleotide in which the quencher is bound to a base of a nucleotide residue.
